# EUROPEAN PATENT SPECIFICATION

(11) **EP 3 164 505 B1**
(45) Date of publication and mention of the grant of the patent: **27.06.2018**
(21) Application number: 15745292.1
(22) Date of filing: 01.07.2015
(51) Int. Cl.: C12Q 1/68

(54) **METHODS FOR LOADING A SENSOR SUBSTRATE**
VERFAHREN ZUR BELADUNG EINES SENSORSUBSTRATS
PROCÉDÉS POUR CHARGER UN SUBSTRAT DE CAPTEUR

(30) Priority: 02.07.2014 US 201462020305 P
(43) Date of publication of application: 10.05.2017
(73) Proprietor: Life Technologies Corporation, Carlsbad, CA 92008 (US)
(72) Inventor: LIGHT, David, Carlsbad, California 92008 (US); CICERO, Ronald L., Carlsbad, California 92008 (US); WANG, Yufang, Carlsbad, California 92008 (US); MASTROIANNI, Alexander, Carlsbad, California 92008 (US); THWAR, Prasanna Krishnan, Carlsbad, California 92008 (US); GRAY, Jeremy, Carlsbad, California 92008 (US); GLAZER, Marc, Carlsbad, California 92008 (US); LINCECUM, Tommie Lloyd Jr., Carlsbad, California 92008 (US); ALANJARY, Mohammad, Escondido, California 92025 (US); KOSCINSKI, Joseph, Carlsbad, California 92008 (US)
(74) Representative: Ludwig, Christine Andrea
(86) International application number: PCT/US2015/038875
(87) International publication number: WO 2016/004234

(56) References cited:
- WO-A1-2014/013263
- US-A1- 2013 225 421
- HOLT ROBERT A ET AL: "The new paradigm of flow cell sequencing", GENOME RESEARCH, COLD SPRING HARBOR LABORATORY PRESS, vol. 18, no. 6, 1 June 2008 (2008-06-01), pages 839-846, XP002666035, ISSN: 1088-9051, DOI: 10.1101/GR.073262.107

## Description

### FIELD OF THE DISCLOSURE

The present disclosure is directed towards the field of molecular biology, in particular towards improved loading and retention of samples onto surfaces, including nucleic acid and protein arrays.

### BACKGROUND

Various techniques for analyzing biomolecules, such as polynucleotides or proteins, rely on the deposition of an array of particles, each attached to such biomolecules. Exemplary sequencing techniques rely on the deposition of an array of particles including a polynucleotide or copies thereof in an array of wells. In a particular example, the particles or beads can be deposited within the wells to associate the particles or beads with a particular sensor and to provide a local environment in which to analyze the biomolecules. In other examples, an ordered array of particles are deposited on a surface and analyzed without the benefit of wells.

### SUMMARY

In an exemplary embodiment, a method of loading beads on a sensor substrate includes applying a suspension including beads to a flow cell defined over a sensor substrate. The sensor substrate includes a plurality of wells. The beads at least partially deposit into the plurality of wells. Holt R.A. et al (The new paradigm of flow cell sequencing, Genome Research vol. 18(6) 2008, p. 839-846) discusses the importance of washing a flow cell in a sequencing reaction. The claimed invention provides an improved method for washing a flow cell that includes removing liquid from the flow cell, evaporating liquid from the flow cell, and applying a hydrating solution to the flow cell.

### BRIEF DESCRIPTION OF THE DRAWINGS

The present disclosure may be better understood, and its numerous features and advantages made apparent to those skilled in the art by referencing the accompanying drawings.
FIG. 1 includes a block flow diagram of an exemplary method for analyzing a target polynucleotide.
FIG. 2 includes a pictorial illustration of an exemplary method for analyzing a target polynucleotide.
FIG. 3 includes a block flow diagram illustrating an exemplary method for loading.
FIG. 4 and FIG. 5 include pictorial illustrations of exemplary methods for loading.
FIG. 6 includes an illustration of an exemplary system for sequencing.
FIG. 7 includes images of a device surface following deposition of beads.
FIG. 8 includes a graph illustrating the intensity ratio of deposited beads.

The use of the same reference symbols in different drawings indicates similar or identical items.

### DETAILED DESCRIPTION

In an exemplary embodiment, a method of loading beads on a sensor substrate includes applying a suspension including beads to a flow cell defined over a sensor substrate. The sensor substrate includes a plurality of reaction sites. Exemplary reaction sites include wells, channels, grooves, pits, dimples, sets of posts, or other similarly functioning structures. The beads at least partially deposit into the plurality of reaction sites. The method also includes removing liquid from the flow cell, evaporating liquid from the flow cell, and applying a hydrating solution to the flow cell. The beads can be conjugated to biomolecules, such as nucleic acids or proteins. The beads can be hydrogel beads. In an example, the sensor substrate can be a semiconductor sequencing device.

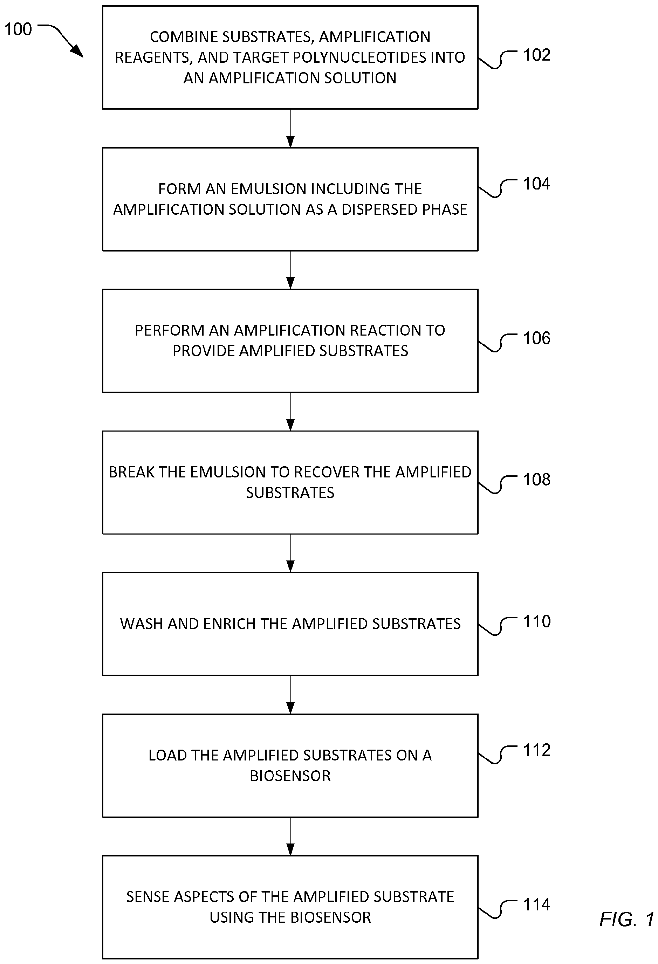

In a particular example, the beads are conjugated with nucleic acids prior to depositing the beads on a sensor substrate including wells. As illustrated in FIG. 1, a method 100 includes combining beads or particles, amplification reagents, and target polynucleotides into an amplification solution, as illustrated at 102. In particular, bead substrates can be formed of hydrophilic polymers. In an example, the beads can carry a charge. Alternatively, the beads can be neutral.

For example, the beads can be formed from monomers including a radically polymerizable monomer, such as a vinyl-based monomer. In an example, the monomer can include acrylamide, vinyl acetate, hydroxyalkylmethacrylate, or any combination thereof. In a particular example, the hydrophilic monomer is an acrylamide, such as an acrylamide including hydroxyl groups, amino groups, carboxyl groups, or a combination thereof. In an example, the hydrophilic monomer is an aminoalkyl acrylamide, an acrylamide functionalized with an amine terminated polypropylene glycol (D, illustrated below), an acrylopiperazine (C, illustrated below), or a combination thereof. In another example, the acrylamide can be a hydroxyalkyl acrylamide, such as hydroxyethyl acrylamide. In particular, the hydroxyalkyl acrylamide can include N-tris(hydroxymethyl)methyl)acrylamide (A, illustrated below), N-(hydroxymethyl)acrylamide (B, illustrated below), or a combination thereof. In a further example, a mixture of monomers, such as a mixture of hydroxyalkyl acrylamide and amine functionalize acrylamide or a mixture of acrylamide and amine functionalized acrylamide, can be used. In an example, the amine functionalize acrylamide can be included in a ratio of hydroxyalkyl acrylamide:amine functionalized acrylamide or acrylamide:amine functionalized acrylamide in a range of 100:1 to 1:1, such as a range of 100:1 to 2:1, a range of 50:1 to 3:1, a range of 50:1 to 5:1 or even a range of 50:1 to 10:1.

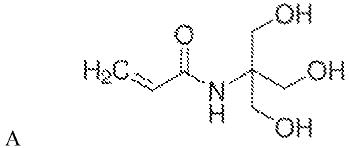

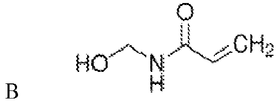

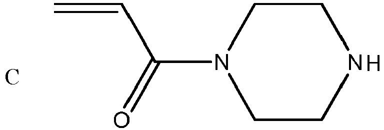

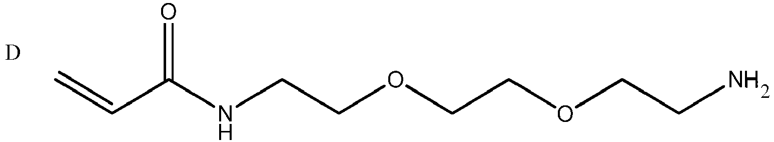

In a particular example, the beads are hydrogel beads.

Each of the beads can include coupling sites to which a template polynucleotide can hybridize. For example, the coupling sites can each include a coupling oligonucleotide complementary to a section of a template polynucleotide. The template polynucleotide can include the target polynucleotide or segments complementary to the target polynucleotide, in addition to segments complementary to the coupling oligonucleotide.

The coupling oligonucleotide can be conjugated to the beads. The polymer of a bead can be activated to facilitate conjugation with a target analyte, such as an oligonucleotide or polynucleotide. For example, functional groups on the beads can be enhanced to permit binding with target analytes or analyte receptors. In a particular example, functional groups of the hydrophilic polymer can be modified with reagents capable of converting the hydrophilic polymer functional groups to reactive moieties that can undergo nucleophilic or electrophilic substitution. For example, hydroxyl groups on the substrate can be activated by replacing at least a portion of the hydroxyl groups with a sulfonate group or chlorine. Exemplary sulfonate groups can be derived from tresyl, mesyl, tosyl, or fosyl chloride, or any combination thereof. Sulfonate can act to permit nucleophiles to replace the sulfonate. The sulfonate can further react with liberated chlorine to provide chlorinated groups that can be used in a process to conjugate the beads. In another example, amine groups on a bead can be activated.

For example, target analyte or analyte receptors can bind to the hydrophilic polymer through nucleophilic substitution with the sulfonate group. In particular example, target analyte receptors terminated with a nucleophile, such as an amine or a thiol, can undergo nucleophilic substitution to replace the sulfonate groups on the surface of the bead.

In another example, sulfonated beads can be further reacted with mono- or multifunctional mono- or multi-nucleophilic reagents that can form an attachment to the beads while maintaining nucleophilic activity for oligonucleotides comprising electrophilic groups, such as maleimide. In addition, the residual nucleophilic activity can be converted to electrophilic activity by attachment to reagents comprising multi-electrophilic groups, which are subsequently to attach to oligonucleotides comprising nucleophilic groups.

In another example, a monomer containing the functional group can be added during the polymerization. The monomer can include, for example, an acrylamide containing a carboxylic acid, ester, halogen or other amine reactive group. The ester group can be hydrolyzed before the reaction with an amine terminated oligonucleotide.

Other conjugation techniques include the use of monomers that comprise amines. The amine is a nucleophilic group that can be further modified with amine reactive bifunctional bis-electrophilic reagents that yield a mono-functional electrophilic group subsequent to attachment to the beads. Such an electrophilic group can be reacted with oligonucleotides having a nucleophilic group, such as an amine or thiol, causing attachment of the oligonucleotide by reaction with the vacant electrophile.

If the beads are prepared from a combination of amino- and hydroxyl-acrylamides, the beads can include a combination of nucleophilic amino groups and neutral hydroxyl groups. The amino groups can be modified with di-functional bis-electrophilic moieties, such as a di-isocyanate or bis-NHS ester, resulting in a hydrophilic particle reactive to nucleophiles. An exemplary bis-NHS ester includes bis-succinimidyl C2-C12 alkyl esters, such as bis-succinimidyl suberate or bis-succinimidyl glutarate.

Other activation chemistries include incorporating multiple steps to convert a specified functional group to accommodate specific desired linkages. For example, a sulfonate modified hydroxyl group can be converted into a nucleophilic group through several methods. In an example, reaction of the sulfonate with azide anion yields an azide substituted hydrophilic polymer. The azide can be used directly to conjugate to an acetylene substituted biomolecule via "CLICK" chemistry that can be performed with or without copper catalysis. Optionally, the azide can be converted to amine by, for example, catalytic reduction with hydrogen or reduction with an organic phosphine. The resulting amine can then be converted to an electrophilic group with a variety of reagents, such as di-isocyanates, bis-NHS esters, cyanuric chloride, or a combination thereof. In an example, using di-isocyanates yields a urea linkage between the polymer and a linker that results in a residual isocyanate group that is capable of reacting with an amino substituted biomolecule to yield a urea linkage between the linker and the biomolecule. In another example, using bis-NHS esters yields an amide linkage between the polymer and the linker and a residual NHS ester group that is capable of reacting with an amino substituted biomolecule to yield an amide linkage between the linker and the biomolecule. In a further example, using cyanuric chloride yields an amino-triazine linkage between the polymer and the linker and two residual chloro-triazine groups one of which is capable of reacting with an amino substituted biomolecule to yield an amino-triazine linkage between the linker and the biomolecule. Other nucleophilic groups can be incorporated into the particle via sulfonate activation. For example, reaction of sulfonated particles with thiobenzoic acid anion and hydrolysis of the consequent thiobenzoate incorporates a thiol into the particle which can be subsequently reacted with a maleimide substituted biomolecule to yield a thio-succinimide linkage to the biomolecule. Thiol can also be reacted with a bromo-acetyl group.

Alternatively, acrydite oligonucleotides can be used during the polymerization to incorporate oligonucleotides. An exemplary acrydite oligonucleotide can include an ion-exchanged oligonucleotides.

Returning to FIG. 1, beads can be incorporated into the amplification solution along with amplification reagents, such as enzymes including polymerase or recombinase, nucleotides (e.g. A, T, C, G, or analogs thereof), various salts or ionic compounds, or a combination thereof. In particular, target polynucleotides, such as polynucleotides derived from biological sources, are included in the amplification solution.

An emulsion is formed that includes the amplification solution as a dispersed phase, as illustrated in 104. In particular, the amplification solution is an aqueous solution and can be dispersed in a hydrophobic phase, such as an oil phase. The hydrophobic phase can include fluorinated liquids, minerals oils, silicone oils, or any combination thereof. Optionally, the hydrophobic phase can include a surfactant, such as a non-ionic surfactant, such as the non-ionic surfactant described below.

The emulsion can be formed utilizing a membrane-based mechanism, in which the aqueous amplification solution and the continuous phase hydrophobic liquid are passed through a membrane one or more times, forming droplets of the amplification solution within the continuous phase hydrophobic liquid. Alternatively, the emulsion can be formed by agitating the amplification solution in the presence of the hydrophobic liquid. In another example, the emulsion can be formed by repeatedly aspirating and ejecting the amplification solution and the hydrophobic continuous phase through a pipette tip. In a further example, droplets of the aqueous amplification solution can be injected into a stream of the hydrophobic liquid.

Following the emulsification of the amplification solution, droplets of amplification solution forming a dispersed phase can include beads and a target polynucleotide. A portion of the droplets can include one or more beads and a single target polynucleotide. Other droplets can include beads and no polynucleotide. Other droplets can include one or more beads and more than one target polynucleotides.

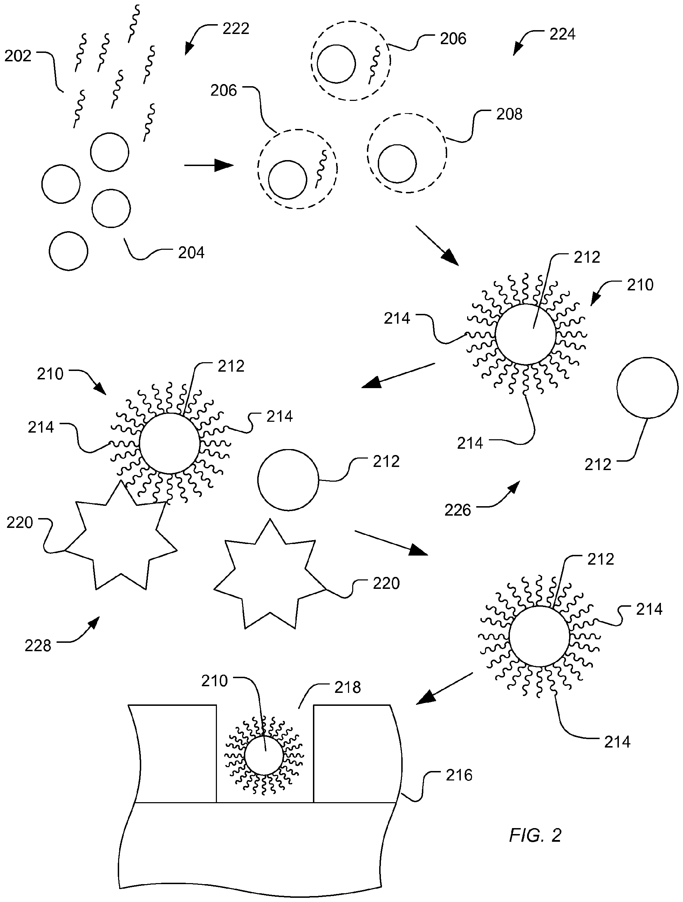

For example, as illustrated in FIG. 2, an aqueous amplification solution 222 includes target polynucleotides 202 and beads 204. Following emulsification, some droplets 206 of the emulsion 224 can include a single target polynucleotide and one or more beads. Other droplets 208 of the emulsion 224 can include a bead and no target polynucleotide.

Returning to FIG. 1, an amplification reaction can be performed to provide nucleic acid beads, as illustrated at 106. The nucleic acid beads include the bead and conjugated copies of nucleic acids. The conditions of the amplification reaction can depend on factors, such as the nature of the enzymes used in the amplification solution, the concentration of individual nucleotides, a concentration of salts or ionic compounds, among other factors. In an example, the amplification reaction is a polymerase chain reaction (PCR) in which the temperature cycles multiple times in a range of 40° C to 100° C. In another example, the amplification reaction is a recombinase polymerase amplification (RPA). Such reactions can be performed isothermally at a temperature in a range of 40° C to 90° C. Other amplification techniques can be used, for example, polymerase cycling assembly (PCA), asymmetric PCR, helicase-dependent amplification, ligation-mediated PCR, multiplex-PCR, nanoparticle-assisted PCR, or other amplification techniques.

In an example, during amplification, template polynucleotides including a target sequence of interest and a segment complementary to the coupling oligonucleotide hybridize to the coupling oligonucleotide. The coupling oligonucleotide is extended, forming a complement to the template polynucleotide. The template polynucleotide can further include a capture moiety useful for binding with a separation substrate for later separation of amplified substrates from unamplified substrates.

As a result of the amplification, dispersed phase droplets including target polynucleotides and beads produce nucleic acid beads including one or more copies of the target polynucleotide conjugated to the beads. In contrast, droplets including a bead and lacking a target polynucleotide do not produce beads that include copies of target polynucleotides and are referred to herein as unamplified bead.

As illustrated at 108, the emulsion is broken to recover nucleic acid beads, whereby the liquid of the dispersed phase is separated from the continuous phase. For example, the emulsion can be applied over a breaking solution and optionally agitated or centrifuged. In particular, centrifuging the emulsion through a breaking solution drives beads into the breaking solution away from an interface between the breaking solution and the continuous phase liquid of the emulsion. The breaking solution can be a hydrophilic liquid, such as an aqueous solution that includes surfactants to assist with augmenting surface tensions and separating the dispersed phase from the continuous phase.

In an example, the breaking solution can include one or more surfactants having a total concentration in the range of 0.01% to 20% by weight. For example, the surfactant can be included in an amount in a range of 0.1% to 15.0%, such as a range of 0.5% to 10.0%, a range of 0.5% to 5.0% or even a range of 0.5% to 3% by weight. In another example, the surfactant can be included in a total amount in a range of 5.0% to 20.0%, such as a range of 10.0% to 20.0%, or a range of 12.0% to 18.0%.

The surfactant can be an ionic surfactant, an amphoteric surfactant, a non-ionic surfactant, or a combination thereof. The ionic surfactant can be an anionic surfactant. An exemplary anionic surfactant includes a sulfate surfactant, a sulfonate surfactant, a phosphate surfactant, a carboxylate surfactant, or any combination thereof. An exemplary sulfate surfactant includes alkyl sulfates, such as ammonium lauryl sulfate, sodium lauryl sulfate (sodium dodecyl sulfate, (SDS)), or a combination thereof; an alkyl ether sulfate, such as sodium laureth sulfate, sodium myreth sulfate, or any combination thereof; or any combination thereof. An exemplary sulfonate surfactant includes an alkyl sulfonate, such as sodium dodecyl sulfonate; docusates such as dioctyl sodium sulfosuccinate; alkyl benzyl sulfonate (e.g., dodecyl benzene sulfonic acid or salts thereof); or any combination thereof. An exemplary phosphate surfactant includes alkyl aryl ether phosphate, alkyl ether phosphate, or any combination thereof. An exemplary carboxylic acid surfactant includes alkyl carboxylates, such as fatty acid salts or sodium stearate; sodium lauroyl sarcosinate; a bile acid salt, such as sodium deoxycholate; or any combination thereof.

In another example, the ionic surfactant can be a cationic surfactant. An exemplary cationic surfactant includes primary, secondary or tertiary amines, quaternary ammonium surfactants, or any combination thereof. An exemplary quaternary ammonium surfactant includes alkyltrimethylammonium salts such as cetyl trimethylammonium bromide (CTAB) or cetyl trimethylammonium chloride (CTAC); cetylpyridinium chloride (CPC); polyethoxylated tallow amine (POEA); benzalkonium chloride (BAC); benzethonium chloride (BZT); 5-bromo-5-nitro-1,3-dioxane; dimethyldioctadecylammonium chloride; dioctadecyldimethylammonium bromide (DODAB); or any combination thereof.

An exemplary amphoteric surfactant includes a primary, secondary, or tertiary amine or a quaternary ammonium cation with a sulfonate, carboxylate, or phosphate anion. An exemplary sulfonate amphoteric surfactant includes (3-[(3-cholamidopropyl) dimethylammonio]-1-propanesulfonate); a sultaine such as cocamidopropyl hydroxysultaine; or any combination thereof. An exemplary carboxylic acid amphoteric surfactant includes amino acids, imino acids, betaines such as cocamidopropyl betaine, or any combination thereof. An exemplary phosphate amphoteric surfactant includes lecithin.

In another example, the surfactant can be a non-ionic surfactant such as a polyethylene glycol-based surfactant, an alkyl pyrrolidine surfactant, an alkyl imidazolidinone surfactant, an alkyl morpholine surfactant, an alkyl imidazole surfactant, an alkyl imidazoline surfactant, or a combination thereof. In a particular example, the polyethylene-glycol-based surfactant includes a polyethylene-glycol ether, such as an alkylphenol polyethoxylate. In another example, the non-ionic surfactant includes a non-ionic fluorosurfactant, such as an ethoxylated fluorocarbon. In a further example, the surfactant solution can include octyl pyrrolidine.

In particular, the surfactant solution can include combinations of such surfactants. For example, the surfactant solution can include a combination of a non-ionic surfactant with an anionic surfactant. In a particular example, the surfactant solution can include a non-ionic surfactant, such as a polyethylene glycol ether, an alkyl pyrrolidine, or a non-ionic fluorosurfactant, and an anionic surfactant, such as a sulfate surfactant, for example SDS. In particular, the surfactant solution can include an ionic surfactant, such as an anionic surfactant, in an amount in a range of 0.1% to 20.0%, such as a range of 1.0% to 15.0%, or a range of 5.0% to 15.0%, or a range of 8.0% to 12.0%. In addition, the surfactant solution can include a non-ionic surfactant, such as alkyl pyrrolidine (e.g., octyl pyrrolidine) in a range of 0.01% to 10.0%, such as a range of 0.05% to 8.0%, or a range of 1.0% to 6.0%. In another example, the surfactant solution can include a non-ionic surfactant in a range of 0.05% to 3.0%.

Referring to FIG. 2, following emulsion breaking, the remaining aqueous solution 226 includes nucleic acid beads 210. The nucleic acid beads 210 can include the beads 212 conjugated to a plurality of copies of the target polynucleotide 214. The solution can also include beads 212 that do not include copies of target polynucleotides, referred to herein as unamplified beads.

Returning to FIG. 1, nucleic acid beads are washed and enriched, as illustrated at 110. In an example, the beads can be pelletized using centrifugation and excess solution can be decanted or drawn from above the pelletized beads. In another example, the nucleic acid beads can be attached to separation substrates used to secure the nucleic acid beads while the aqueous solution surrounding the nucleic acid beads is replaced.

For example, as illustrated in FIG. 2, nucleic acid beads 210 can be captured by a separation substrate 220. In contrast, the unamplified beads 212 that do not include copies of polynucleotides do not readily attached to the separation substrate 220. Thus, when the separation substrate 220 is secured and the attached nucleic acid beads 210 are held in place, the unsecured unamplified beads are substantially washed from the solution. In a particular example, the separation substrates 214 are magnetic substrates that can be secured to a container wall using a magnetic field. The nucleic acid beads 210 can then be separated from the separation substrates 220 providing a solution that has predominantly nucleic acid beads and substantially fewer unamplified beads.

In a particular example, the nucleic acid beads 210 can include a capture moiety that interacts with moieties on the separation substrates 220. The unamplified beads can be substantially free of the capture moieties. For example, the template polynucleotide can be terminated with a capture moiety. The unamplified beads not hybridized to a template polynucleotide lack the capture moiety and thus, do not bind with the separation substrate. Once the nucleic acid beads 210 are separated from the unamplified beads, the nucleic acid beads 210 can be separated from the separation substrate, for example, by melting or detaching the template polynucleotide from the extended coupling oligonucleotides conjugated to the nucleic acid beads 210.

Returning to FIG. 1, the enriched nucleic acid beads can be loaded onto a biosensor, as illustrated at 112. Depending upon the nature of the biosensor, the biosensor can provide a surface onto which the nucleic acid beads can be attached. The surface can be flat and optionally can include regions that are more attractive to the nucleic acid beads or that are modified to secure the nucleic acid beads. In another example, the biosensor can include a surface that includes discrete sites or patterned surfaces, such as dimples, depressions, pores, wells, ridges or channels into which the nucleic acid beads align. In a further example, as illustrated in FIG. 2, the biosensor can include a surface structure 216 that defines a well 218 into which the nucleic acid beads 210 are deposited.

In a particular example, the well is defined over a sensor. The well walls or the sensor can have surfaces formed of metals, semi-metals, oxides thereof, nitrides thereof, or a combination thereof. In an example, the well wall can be formed of a semi-metal, such as silicon, an oxide thereof such as silicon dioxide, a nitride thereof such as silicon nitride, or a combination thereof. In another example, the wall of the well can be formed at least partially of a metal or metal oxide. Exemplary metals include titanium, tungsten, tantalum, hafnium, aluminum, zirconium, zinc, or a combination thereof. A portion of the well wall can be formed of an oxide or nitride of such metals, such as, for example, titanium oxide, tantalum oxide, hafnium oxide, aluminum oxide, zirconium oxide, titanium nitride, among others, or a combination thereof. Further, the sensor forming a bottom of the well can have a metal, metal oxide, or metal nitride surface, or a combination thereof. Exemplary metals include titanium, tungsten, tantalum, hafnium, aluminum, zirconium, or zinc, or a combination thereof. Exemplary metal oxides or nitrides include titanium oxide, tantalum oxide, hafnium oxide, aluminum oxide, zirconium oxide, titanium nitride, among others, or a combination thereof.

In a particular example, the walls of the well or a surface of the sensor can have hydroxyl groups, for example, in a concentration in a range of 8 to 20 OH/nm², such as between 8 and 16 OH/nm². Optionally, the surface can be treated with a sulfate, phosphate, or silane containing compound. The compound can bind to at least a portion of the OH groups residing on the surface of the well wall or sensor. In a particular example, the compound includes a silane containing compound, such as butyl ammonium trimethoxy silane (BATS) or a phosphonic acid containing compound, such as an imidazole alkyl phosphonic acid, e.g., (1-methyl-3- (dodecylphosphonic acid) imidazolium bromide) (ImPA).

As illustrated at 114 of FIG. 1, the biosensor can sense aspects of the nucleic acid beads. Depending upon the nature of the biosensor, the sensor can be utilized to detect the presence of particular sequences within the target polynucleotide, or can be used to sequence the target polynucleotide. For example, the biosensor can utilize fluorescence-based sequencing-by-synthesis. In another example, the biosensor can utilize techniques for sequencing that include sensing byproducts of nucleotide incorporation, such as pH or the presence of pyrophosphate or phosphate. In a further example, the biosensor can utilize temperature or heat detection.

Returning to FIG. 2, in an example, a well 218 of an array of wells can be operatively connected to measuring devices. For example, for fluorescent emission methods, a well 218 can be operatively coupled to a light detection device. In the case of ionic detection, the lower surface of the well 218 can be disposed over a sensor pad of an ionic sensor, such as a field effect transistor.

One exemplary system involving sequencing via detection of ionic byproducts of nucleotide incorporation includes semiconductor sequencing platforms, such as an ion-based sequencing system that sequences nucleic acid templates by detecting hydrogen ions produced as a byproduct of nucleotide incorporation. Typically, hydrogen ions are released as byproducts of nucleotide incorporations occurring during template-dependent nucleic acid synthesis by a polymerase. Such a sequencer detects the nucleotide incorporations by detecting the hydrogen ion byproducts of the nucleotide incorporations. Such a sequencer can include a plurality of template polynucleotides to be sequenced, each template disposed within a respective sequencing reaction well in an array. The wells of the array can each be coupled to at least one ion sensor that can detect the release of H+ ions or changes in solution pH produced as a byproduct of nucleotide incorporation. The ion sensor comprises a field effect transistor (FET) coupled to an ion-sensitive detection layer that can sense the presence of H+ ions or changes in solution pH. The ion sensor can provide output signals indicative of nucleotide incorporation which can be represented as voltage changes whose magnitude correlates with the H+ ion concentration in a respective well or reaction chamber. Different nucleotide types can be flowed serially into the reaction chamber, and can be incorporated by the polymerase into an extending primer (or polymerization site) in an order determined by the sequence of the template. Each nucleotide incorporation can be accompanied by the release of H+ ions in the reaction well, along with a concomitant change in the localized pH. The release of H+ ions can be registered by the FET of the sensor, which produces signals indicating the occurrence of the nucleotide incorporation. Nucleotides that are not incorporated during a particular nucleotide flow may not produce signals. The amplitude of the signals from the FET can also be correlated with the number of nucleotides of a particular type incorporated into the extending nucleic acid molecule thereby permitting homopolymer regions to be resolved. Thus, during a run of the sequencer multiple nucleotide flows into the reaction chamber along with incorporation monitoring across a multiplicity of wells or reaction chambers can permit the instrument to resolve the sequence of many nucleic acid templates simultaneously.

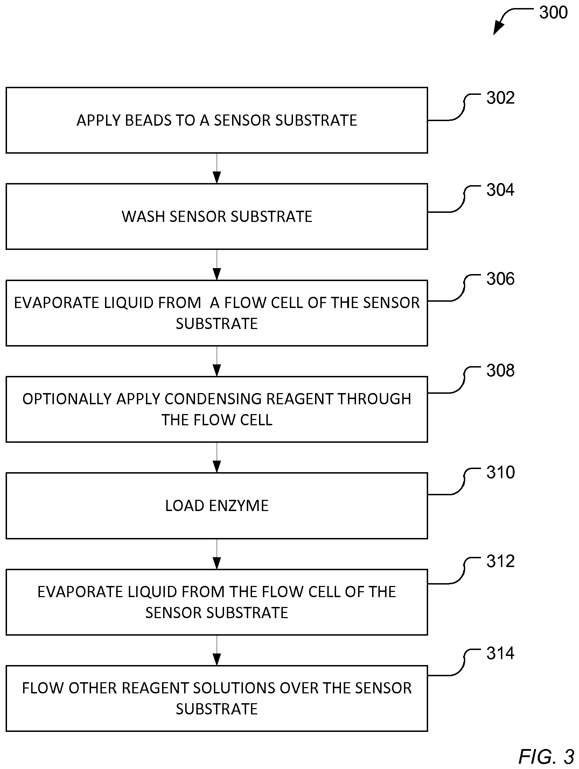

In a particular example, the biosensor includes a sensor substrate and a flow cell defined over the sensor substrate. The nucleic acid beads can be applied to the sensor substrate to deposit into the wells of the sensor substrate. The beads can be exposed to gas flowing through the flow cell, optionally exposed to a condensing reagent, and undergo additional processing before being applied to a detection system that utilizes the biosensor, such as a sequencing system. For example, as illustrated in FIG. 3, a method 300 includes applying beads to a sensor substrate, as illustrated at 302. A suspension including the beads and optionally salts, buffering agents, or surfactants, can be applied through the flow cell. Optionally, the biosensor can be vortexed or centrifuged to further encourage the beads to deposit within the wells of the sensor substrate. In a particular example, the biosensor is centrifuged at an angle relative to a plane of rotation of at least 30°, such as an angle between 30 and 90° with the well openings generally facing inward toward the axis of rotation. The biosensor including the suspension within the flow cell can be centrifuged for a period ranging between 30 seconds and 15 minutes, such as a period ranging between 1 minute and 10 minutes.

As illustrated at 304 of FIG. 3, the sensor substrate can be washed, flushing the suspension from the flow cell and leaving beads deposited in wells of the sensor substrate. In an example, during washing, a gas/liquid interface can flow over the substrate. The gas/liquid interface can be applied by intermittently flowing gas and liquid through the flow cell, forming bubbles. In another example, a foam can be formed that is applied through the flow cell causing a plurality of gas/liquid interfaces to flow over the wells of the sensor substrate.

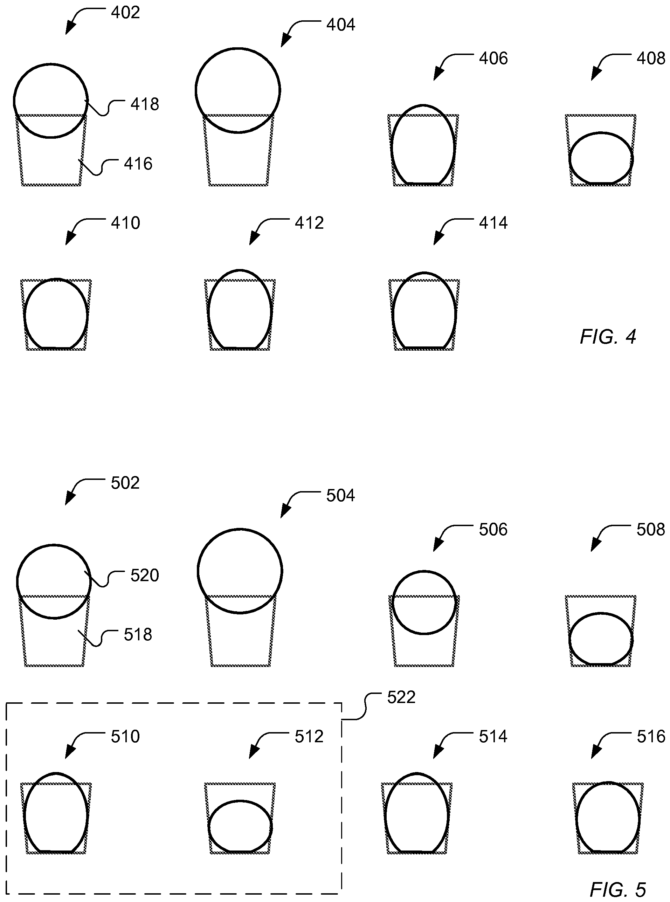

As illustrated in FIG. 4, when the suspension including beads is applied over the sensor substrate, the beads 418 partially deposit within the wells 416, as illustrated at 402. Following wash and optional application of the gas/liquid interfaces, as illustrated at 404, the bead can swell, but remain partially engaged within the well 416.

As illustrated at 306 of FIG. 3, gas can be applied through the flow cell, evaporating liquid out of the flow cell. The gas can be pushed through the flow cell. Alternatively, the gas can be drawn through the flow cell to evaporate liquid. When pushing gas through the flow cell, pressure can increases within the flow cell relative to atmospheric pressure. When drawing gas through the flow cell, pressure within the flow cell can decreases relative to atmospheric pressure. It is believed that flowing the gas through the flow cell and particularly drawing the gas through the flow cell further drives the beads into the wells and partially dries the wells, evaporating liquid from the wells. Alternatively, the liquid can be spun out of the flow cell using centrifugation. The gas can be nitrogen, a noble gas, such as helium or argon, or air. As illustrated in FIG. 4 at 406, the flow of gas through the flow cell, particularly drawing gas through the flow cell, further draws the beads into the wells.

In an example, the gas can flow through the flow cell, such as being drawn through the flow cell, for a period of at least 15 seconds, such as a period of at least 30 seconds, at least 45 seconds, at least a minute, at least 1.5 minutes, at least 2 minutes, or even at least 5 minutes. In general, gas does not flow through the flow cell longer than 30 minutes, such as not longer than 20 minutes. The gas can be applied at a rate in a range of 100 µL/min to 100 mL/min, such as a range of 500 µL/min to 60 mL/min, a range of 500 µL/min to 40 mL/min, or even a range of 1 mL/min to 20 mL/min.

Optionally, a condensing agent can be applied over the sensor substrate following the flow of gas, as illustrated at 308 of FIG. 3. The condensing reagent can decrease the diameter of the beads further pushing the beads within the well. As illustrated in FIG. 4 at 408, application of the condensing reagent causes the beads to shrink and draw further within the wells.

An exemplary condensing reagent includes a condensing agent, such as a metal complex, an alkali or alkali-earth metal salt, a non-ionic polymer, an alcohol, or a combination thereof. In an example, the condensing agent includes a metal-complex including cobalt, for example, forming a cobalt organic complex, such as a cobalt-amine complex.

Alternatively or additionally, the condensing reagent can include a condensing agent that influences the density of the polymer matrix of the bead. For example, the solution can include an alcohol, such as methanol, ethanol or isopropyl alcohol (IPA), which can influence the density of the polymer matrix of the bead. In particular, the solution can include an alcohol, such as methanol, in a concentration in a range of 0.1 vol.% to 60 vol.%, such as a range of 0.1 vol.% to 50 vol.%, a range of 0.1 vol.% to 30 vol.%, a range of 0.1 vol.% to 20 vol.%, or even a range of 0.1 vol.% to 10 vol.%. For example, the concentration of alcohol within the solution can be in a range of 0.1 vol.% to 5 vol.%, such as 1 vol.% to 5 vol.%. In another example, the solution can include an alcohol, such as ethanol or IPA in a concentration in a range of 0.1 vol.% to 60 vol.%, such as a range of 1.0 vol.% to 60 vol.%, a range of 5.0 vol.% to 60 vol.%, a range of 10 vol.% to 60 vol.%, or even a range of 40 vol.% to 60 vol.%.

In another example, the condensing agent includes concentrated alkali or alkali-earth metal salts, such as halide salts. In an additional example, the condensing agent can include magnesium chloride, for example, in a concentration in a range of 20 mM to 1M, such as a range of 100 mM to 800 mM, or even a range of 100 mM to 500 mM.

The condensing agent can further be a non-ionic polymer, such as a polyethylene glycol based polymer. In a particular example, the polyethylene glycol based polymer has a molecular weight in a range between 2000 and 20000, such as between 5000 and 15000, or between 8000 and 12000. The non-ionic polymer can be included in a concentration in a range of 0.1 wt% to 20.0 wt%, such as a range of 0.5 wt% to 15.0 wt%, a range of 0.5 wt% to 10 wt%, or a range of 2.5 wt% to 7.5 wt%.

The remainder of the condensing reagent can include a buffered solution, such as buffered saline solution. For example, the remainder of the condensing reagent can include a phosphate buffered saline solution. In particular, the solution can include sodium or potassium halide salts, sodium or potassium phosphate salts, or polysorbate. Such salts can be included, for example, in amounts in a range of 1 mM to 500 mM, such as a range of 50 mM to 350 mM, or a range of 150 mM to 250 mM. In an example, potassium chloride can be included in a concentration in a range of 0.5 M to 2 M, such as a range of 0.8M to 1.5M, or even a range of 0.8M to 1.2M. In addition or alternatively, other buffering agents can be used, such as an amine based buffering agent, e.g., tris(hydroxymethyl)aminomethane. Such an amine buffering agent can be used in a concentration in a range of 10 mM to 1M, such as a range of 100mM to 1M, a range of 100mM to 800mM, or even a range of 150 mM to 500 mM. Optionally, the condensing reagent can include other ionic components, such as calcium or magnesium, derived from salts. Further, the solution can have a pH between 6 and 9, such as between 6.5 and 8.5, or between 7 and 8.5.

The condensing reagent can include a surfactant. For example, the surfactant can be a non-ionic polymer surfactant, such as an ether of polyethylene glycol, for example an octylphenyl ether of polyethylene glycol. The non-ionic polymer surfactant can be included in a range of 0.01% to 1.0%, such as a range of 0.05% to 0.8%, a range of 0.05% to 0.5%, or even a range of 0.08% to 0.15%. An exemplary surfactant is TritonX-100.

In an example, a condensing reagent includes a condensing agent, such as MgCl₂ in a range of 20 mM to 500 mM; a salt, such as KCl in a range of 0.8M to 1M; a buffering agent, such as tris(hydroxymethyl)aminomethane in a range of 150 mM to 500 mM; and a surfactant, such as TritonX-100 in a range of 0.05% to 0.5%. The pH is in a range of 7 to 9.

In another example, the a condensing reagent includes a combination of condensing agents, such as MgCl₂ in a range of 20 mM to 500 mM and polyethylene glycol in a range of 0.5wt% to 10.0 wt%. The polyethylene glycol can have a molecular weight in a range of 1000 to 15000. The condensing reagent can also include a salt, such as KCl in a range of 0.8M to 1M; a buffering agent, such as tris(hydroxymethyl)aminomethane in a range of 10 mM to 500 mM; or a surfactant, such as TritonX-100 in a range of 0.05% to 0.5%. The pH is in a range of 7 to 9.

In response to exposure to the condensing reagent, the nucleic acid beads including the nucleic acid strands can decrease in diameter. For example, the bead or particle diameter can decrease by at least 1% in response to exposure to the solution. In an example, the diameter decreases by at least 5%, such as at least 10%, at least 15%, or even at least 19%. In a particular example, the diameter decreases by not greater than 75%. Further, the density of the bead or particle can increase in response to exposure to a condensing agent. For example, the density can increase by at least 2%, such as at least 8%, at least 14%, at least 21%, at least 25%, at least 50%, at least 65%, or even at least 80%. In particular, the density increases by not greater than 7500%.

Optionally, the process of washing the sensor substrate, as illustrated at 304 of FIG. 3, flowing gas through the flow cell, as illustrated at 306, or applying a condensing reagent, as illustrated at 308, can be repeated in total or in part one or more times.

In a case of a sequencing application or other biomolecular applications that involve the use of enzymes, enzyme can be loaded onto the beads deposited on the sensor substrate, as illustrated at 310. In particular, a solution including an enzyme can be applied through the flow cell and contacted with the beads deposited within the wells. In general, such contact with enzymes causes an increase in the dynamic diameter or size of beads.

Optionally, after loading the enzyme, the process of flowing gas through the flow cell can be performed again using flow rates and periods as described above, as illustrated at 312, for example, to evaporate liquid from the flow cell. As enzymes are loaded, the beads increase in size. For example, as illustrated in FIG. 4 at 410, loading the enzyme can result in an initial swelling of the bead and further incubation in the presence of the enzyme can cause further swelling of the bead, as illustrated at 412. Further drawing gas through the flow cell can partially draw the beads back into the well after the bead swells in response to incubation in the presence of enzyme, as illustrated at 414 of FIG. 4.

As illustrated at 314 of FIG. 3, additional reagents or other solutions can flow through the flow cell either to prepare the sensor substrate for further processing or as part of a testing routine. For example, reagent solutions including nucleotides can flow over the sensor substrate during a sequencing reaction.

In a further exemplary method illustrated in FIG. 5, the beads 520 can initially be spun into wells 518 on the sensor substrate, as illustrated at 502. The beads 520 can be washed, as illustrated at 504, and optionally a gas/liquid interfaces (e.g., in the form of a foam) can be applied to the sensor substrate, initially increasing the size of the bead 520. The condensing agent can be applied as illustrated at 506, and the liquid can be removed and then liquid can be evaporated, as illustrated at 508, to shrink and drive the bead 520 deeper into the well 518. Removing the liquid can include applying an air bubble to drive liquid from the flow cell or spinning the flow cell to force liquid out and draw air in. Evaporating liquid from the flow cell can be accomplished by drawing air or dry nitrogen through the flow cell of the sensor substrate or pushing air or nitrogen through the flow cell.

After removing the liquid, the beads 520 can be washed, as illustrated at 510. Condensing agent can be applied again and the liquid evaporated (e.g., gas applied), as illustrated at 512. Wash, condensing, and evaporating can be repeated between 0 and 2 times (522).

As illustrated at 514, enzymes can be loaded into the beads 520 and optionally the beads can be washed and gas applied, as illustrated at 516.

Embodiments of the above described methods for loading beads onto a sensor substrate including wells provide the technical advantage of drawing beads, particularly those conjugated to nucleic acids or proteins, into the wells of the sensor substrate. Better seating and deposition of such beads into wells provide an increase in key signal or a decrease in signal-to-noise ratios. Such a process is further particularly suited for systems incorporating hydrogel beads and systems utilizing well walls or sensor surfaces that exhibit OH groups on the surface. Even in cases in which such OH groups are partially blocked by surface coatings, embodiments of the above-described methods provide for improved loading, higher key signal, or higher signal-to-noise.

As described above, the nucleic acid beads can be loaded into a biosensor for determining characteristics of the nucleic acid beads. In particular, the nucleic acid beads can be used for sequence target sequences conjugated to the nucleic acid beads. For example, sequencing can include label-free DNA sequencing, and in particular, pH-based DNA sequencing. Substrates including DNA templates and having a primer and polymerase operably bound are loaded into reaction chambers (such as microwells), after which repeated cycles of deoxynucleoside triphosphate (dNTP) addition and washing are carried out. Such templates are typically attached as clonal populations to the substrate, such as a microparticle, bead, or the like, and such clonal populations are loaded into reaction chambers. In each addition step of the cycle, the polymerase extends the primer by incorporating added dNTP when the next base in the template is the complement of the added dNTP. When there is one complementary base, there is one incorporation, when two, there are two incorporations, when three, there are three incorporations, and so on. With each such incorporation there is a hydrogen ion released, and collectively a population of templates releasing hydrogen ions causes very slight changes the local pH of the reaction chamber, which is detected by an electronic sensor.

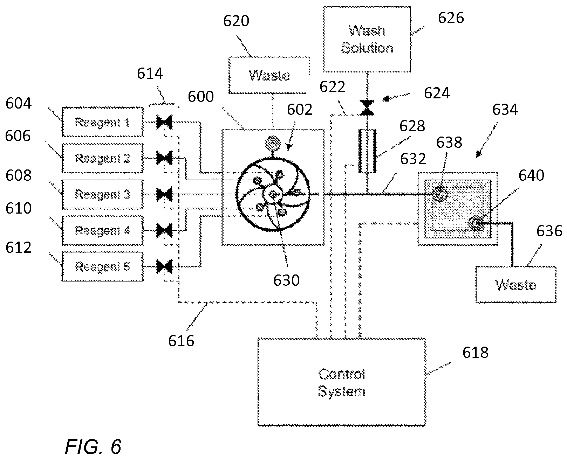

FIG. 6 diagrammatically illustrates an apparatus for carrying out pH-based nucleic acid sequencing. Each electronic sensor of the apparatus generates an output signal that depends on the value of a reference voltage. In FIG. 6, housing (600) containing fluidics circuit (602) is connected by inlets to reagent reservoirs (604, 606, 608, 610, and 612), to waste reservoir (620) and to flow cell (634) by passage (632) that connects fluidics node (630) to inlet (638) of flow cell (634). Reagents from reservoirs (604, 606, 608, 610, and 612) can be driven to fluidic circuit (602) by a variety of methods including pressure, pumps, such as syringe pumps, gravity feed, and the like, and are selected by control of valves (614). Control system (618) includes controllers for valves (614) that generate signals for opening and closing via electrical connection (616). Control system (618) also includes controllers for other components of the system, such as wash solution valve (624) connected thereto by (622), and reference electrode (628). Control system (618) can also include control and data acquisition functions for flow cell (634). In one mode of operation, fluidic circuit (602) delivers a sequence of selected reagents (1, 2, 3, 4, or 5) to flow cell (634) under programmed control of control system (618), such that in between selected reagent flows fluidics circuit (602) is primed and washed, and flow cell (634) is washed. Fluids entering flow cell (634) exit through outlet (640) and are deposited in waste container (636). Throughout such an operation, the reactions or measurements taking place in flow cell (634) have a stable reference voltage because reference electrode (628) has a continuous, i.e. uninterrupted, electrolyte pathway with flow cell (634), but is in physical contact with only the wash solution.

### EXAMPLE

Hydrogel polyacrylamide beads conjugated to nucleic acids are deposited on a sensor substrate having a well diameter of 0.7 µm. The wells are formed of a silicon nitride wall. The underlying sensor pad and a portion of the wall are formed of a titanium surface treated with ImPA. The beads have an average diameter of approximately 0.68 µm and are stained with a SYBR Gold fluorescent dye available from Life Technologies Corporation.

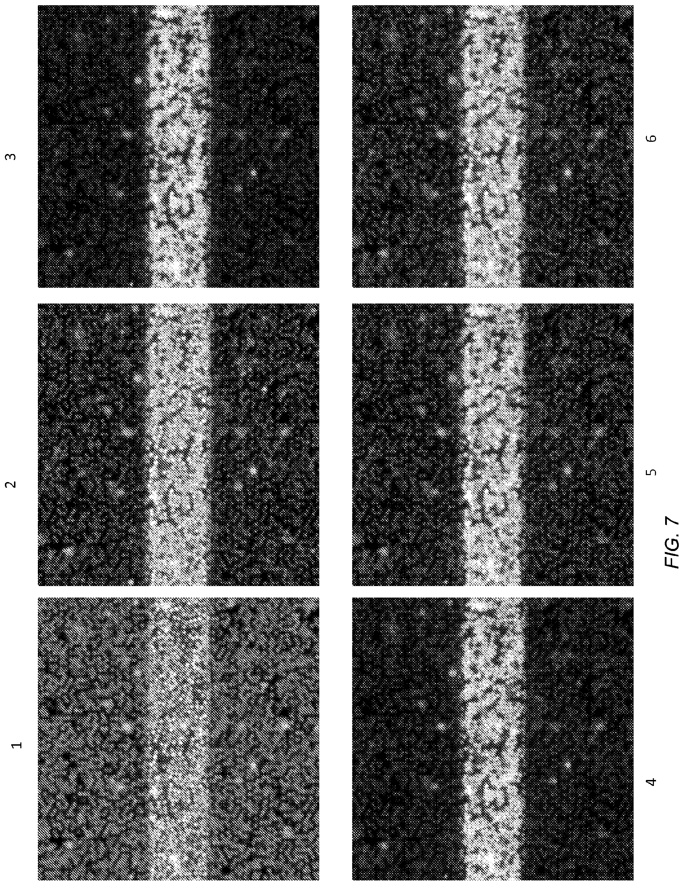

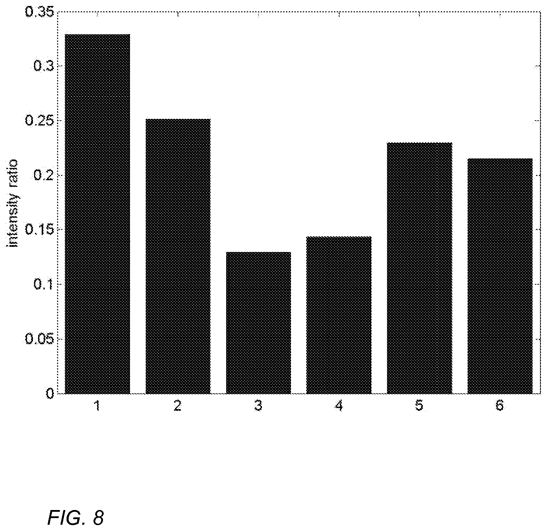

A suspension including the beads is applied over the substrate surface to at least partially deposit beads within wells, as illustrated in FIG. 7, image 1. The image includes a brighter central strip that is free of wells and represents beads deposited on an interstitial surface. Beads deposited on either side of the center strip having a lower intensity are at least partially deposited within wells. The ratio of the average intensity of the beads within a well to that of a bead on the interstitial center strip indicates the depth within a well a bead is deposited. As illustrated in FIG. 8 bar 1, the average bead has an intensity ratio (ratio of the average deposited bead intensity to that of the bead on interstitial surface) of between 0.3 and 0.35. Following the drawing of gas through the flow cell to evaporate liquid from the wells, the average bead deposited within the wells exhibits a lower intensity ratio, as illustrated in FIG. 7, image 2, and FIG. 8, bar 2. Such a reduction in the intensity ratio represents a further drawing of the bead into the wells.

The beads are further treated with a condensing reagent that includes 100 mmol magnesium chloride and 5 wt% polyethylene glycol having a molecular weight of approximately 10,000. As illustrated at FIG. 7 image 3 and FIG. 8 bar 3, following treatment with the condensing agent, the intensity ratio falls significantly.

The beads can be further spun on a centrifuge in the presence of the condensing reagent, as illustrated at FIG. 7 image 4 or FIG. 8 bar 4. Such spinning in the presence of the condensing agent has limited influence on the intensity ratio and can actually increase the intensity ratio.

The beads can be incubated in the presence of enzyme. As illustrated at FIG. 7 image 5 and FIG. 8 bar 5, such incubation in the presence of enzyme causes a swelling of the bead and thus an increase in the intensity ratio. Gas can be further drawn through the flow cell following enzyme loading to evaporate liquid from the wells, resulting in beads having slightly lower intensity ratios, as illustrated at FIG. 7 image 6 and FIG. 8 bar 6.

In a first aspect, a method of loading beads on a sensor substrate includes applying a suspension including beads to a flow cell defined over a sensor substrate, the sensor substrate comprising a plurality of wells, the beads at least partially depositing into the plurality of wells; removing liquid from the flow cell; evaporating liquid from the flow cell; and applying a hydrating solution to the flow cell.

In an example of the first aspect, applying the suspension includes centrifuging the sensor substrate in the presence of the beads.

In another example of the first aspect and the above examples, the method further includes flowing one or more bubbles through the flow cell and over the sensor substrate prior to evaporating the liquid from the flow cell.

In a further example of the first aspect and the above examples, evaporating liquid from the flow cell includes drawing gas through the flow cell for a period of at least 15 seconds and not longer than 30 minutes.

In an additional example of the first aspect and the above examples, evaporating liquid from the flow cell includes drawing gas through the flow cell at a rate in a range of 100 (µL/min to 100 mL/min.

In another example of the first aspect and the above examples, the method further includes applying a condensing solution over the sensor substrate. For example, applying the condensing solution includes applying the condensing solution after evaporating liquid from the flow cell. In an example, applying the condensing solution includes applying the condensing solution before evaporating liquid from the flow cell. In another example, the condensing solution includes a condensing agent. For example, the condensing agent includes magnesium, a polyethylene glycol polymer, or a combination thereof.

In a further example of the first aspect and the above examples, the method further includes applying an enzyme solution through the flow cell and over the sensor substrate. For example, the method further includes removing liquid from the flow cell and evaporating liquid from the flow cell after applying the enzyme solution.

In an additional example of the first aspect and the above examples, the sensor substrate includes a semiconductor sequencing device. In an example, the semiconductor sequencing device includes an array of pH sensors. For example, the method further includes sequencing using the semiconductor sequencing device.

In another example of the first aspect and the above examples, the beads include hydrogel beads.

In a second aspect, a method of loading beads on a sensor substrate includes applying a suspension including nucleic acid beads to a flow cell defined over a sensor substrate, the sensor substrate comprising a plurality of wells, the beads at least partially depositing into the plurality of wells; flowing a gas/liquid interface through the flow cell and over the sensor substrate; evaporating liquid from the flow cell for a period of at least 15 seconds and not longer than 30 minutes at a rate in a range of 100 µL/min to 100 mL/min; and applying a condensing solution through the flow cell.

In an example of the second aspect, the condensing solution includes a magnesium salt and a polyethylene glycol polymer.

In another example of the second aspect and the above examples, flowing the gas/liquid interface includes flowing a foam through the flow cell.

In a further example of the second aspect and the above examples, the method further includes applying an enzyme solution through the flow cell after applying the condensing solution

Note that not all of the activities described above in the general description or the examples are required, that a portion of a specific activity may not be required, and that one or more further activities can be performed in addition to those described. Still further, the order in which activities are listed are not necessarily the order in which they are performed.

In the foregoing specification, the concepts have been described with reference to specific embodiments. Accordingly, the specification and figures are to be regarded in an illustrative rather than a restrictive sense.

As used herein, the terms "comprises," "comprising," "includes," "including," "has," "having" or any other variation thereof, are intended to cover a non-exclusive inclusion. For example, a process, method, article, or apparatus that comprises a list of features is not necessarily limited only to those features but may include other features not expressly listed or inherent to such process, method, article, or apparatus. Further, unless expressly stated to the contrary, "or" refers to an inclusive-or and not to an exclusive-or. For example, a condition A or B is satisfied by any one of the following: A is true (or present) and B is false (or not present), A is false (or not present) and B is true (or present), and both A and B are true (or present).

Also, the use of "a" or "an" are employed to describe elements and components described herein. This is done merely for convenience and to give a general sense of the scope of the invention. This description should be read to include one or at least one and the singular also includes the plural unless it is obvious that it is meant otherwise.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

After reading the specification, skilled artisans will appreciate that certain features are, for clarity, described herein in the context of separate embodiments, may also be provided in combination in a single embodiment. Conversely, various features that are, for brevity, described in the context of a single embodiment, may also be provided separately or in any subcombination. Further, references to values stated in ranges include each and every value within that range.

## Claims

1. A method of loading beads on a sensor substrate, the method comprising:
applying a suspension including beads to a flow cell defined over a sensor substrate, the sensor substrate comprising a plurality of wells, the beads at least partially depositing into the plurality of wells;
removing liquid from the flow cell;
evaporating liquid from the flow cell; and
applying a hydrating solution to the flow cell.

2. The method of claim 1, wherein applying the suspension includes centrifuging the sensor substrate in the presence of the beads.

3. The method of claim 1 or claim 2, further comprising flowing one or more bubbles through the flow cell and over the sensor substrate prior to removing the liquid from the flow cell.

4. The method of any one of claims 1-3, wherein evaporating liquid from the flow cell includes drawing gas through the flow cell for a period of at least 15 seconds and not longer than 30 minutes.

5. The method of any one of claims 1-4, wherein evaporating liquid from the flow cell includes drawing gas through the flow cell at a rate in a range of 100 (µL/min to 100 mL/min.

6. The method of any one of claims 1-5, further comprising applying a condensing solution over the sensor substrate.

7. The method of claim 6, wherein applying the condensing solution includes applying the condensing solution after drawing gas through the flow cell.

8. The method of claim 6, wherein applying the condensing solution includes applying the condensing solution before evaporating liquid from the flow cell.

9. The method of claim 6, wherein the condensing solution includes a condensing agent, optionally wherein the condensing agent includes magnesium, a polyethylene glycol polymer, or a combination thereof.

10. The method of any one of claims 1-9, further comprising applying an enzyme solution through the flow cell and over the sensor substrate.

11. The method of claim 10, further comprising removing liquid from the flow cell and evaporating liquid from the flow cell after applying the enzyme solution.

12. The method of any one of claims 1-11, wherein the sensor substrate includes a semiconductor sequencing device, optionally wherein the semiconductor sequencing device includes an array of pH sensors, and optionally wherein the method further comprises sequencing using the semiconductor sequencing device.

13. A method of loading beads on a sensor substrate, the method comprising:
applying a suspension including nucleic acid beads to a flow cell defined over a sensor substrate, the sensor substrate comprising a plurality of wells, the beads at least partially depositing into the plurality of wells;
flowing a gas/liquid interface through the flow cell and over the sensor substrate;
evaporating liquid from the flow cell for a period of at least 15 seconds and not longer than 30 minutes at a rate in a range of 100 (µL/min to 100 mL/min; and
applying a condensing solution through the flow cell.

14. The method of claim 13, wherein the condensing solution includes a magnesium salt and a polyethylene glycol polymer.

15. The method of claim 13 or claim 14, wherein flowing the gas/liquid interface includes flowing a foam through the flow cell.

## Patentansprüche

1. Verfahren zum Laden von Kügelchen auf ein Sensorsubstrat, das Verfahren umfassend:
Zufügen einer Suspension, die Kügelchen beinhaltet, zu einer über einem Sensorsubstrat definierten Durchflusszelle, wobei das Sensorsubstrat eine Vielzahl von Mulden umfasst, wobei die Kügelchen sich mindestens teilweise in der Vielzahl von Mulden absetzen;
Entfernen von Flüssigkeit von der Durchflusszelle;
Verdampfen von Flüssigkeit von der Durchflusszelle; und
Zufügen einer hydratisierenden Lösung zur Durchflusszelle.

2. Verfahren nach Anspruch 1, wobei das Zufügen der Suspension Zentrifugieren des Sensorsubstrats in Gegenwart der Kügelchen beinhaltet.

3. Verfahren nach Anspruch 1 oder Anspruch 2, ferner umfassend Strömenlassen von einer oder mehreren Luftblasen durch die Durchflusszelle und über das Sensorsubstrat vor dem Entfernen der Flüssigkeit von der Durchflusszelle.

4. Verfahren nach einem der Ansprüche 1-3, wobei das Verdampfen von Flüssigkeit von der Durchflusszelle Ziehen von Gas durch die Durchflusszelle für eine Dauer von mindestens 15 Sekunden und nicht länger als 30 Minuten beinhaltet.

5. Verfahren nach einem der Ansprüche 1-4, wobei das Verdampfen von Flüssigkeit von der Durchflusszelle Ziehen von Gas durch die Durchflusszelle mit einer Rate in einem Bereich von 100 µl/min bis 100 ml/min beinhaltet.

6. Verfahren nach einem der Ansprüche 1-5, ferner umfassend Aufbringen einer Kondensationslösung über dem Sensorsubstrat.

7. Verfahren nach Anspruch 6, wobei das Aufbringen der Kondensationslösung Aufbringen der Kondensationslösung nach dem Ziehen von Gas durch die Durchflusszelle beinhaltet.

8. Verfahren nach Anspruch 6, wobei das Aufbringen der Kondensationslösung Aufbringen der Kondensationslösung vor dem Verdampfen von Flüssigkeit von der Durchflusszelle beinhaltet.

9. Verfahren nach Anspruch 6, wobei die Kondensationslösung ein Kondensationsmittel beinhaltet, wobei das Kondensationsmittel wahlweise Magnesium, ein Polyethylenglykolpolymer, oder eine Kombination davon beinhaltet.

10. Verfahren nach einem der Ansprüche 1-9, ferner umfassend das Aufbringen einer Enzymlösung durch die Durchflusszelle und über dem Sensorsubstrat.

11. Verfahren nach Anspruch 10, ferner umfassend das Entfernen von Flüssigkeit von der Durchflusszelle und das Verdampfen von Flüssigkeit von der Durchflusszelle nach dem Aufbringen der Enzymlösung.

12. Verfahren nach einem der Ansprüche 1-11, wobei das Sensorsubstrat eine Halbleitersequenzierungsvorrichtung beinhaltet, wobei die Halbleitersequenzierungsvorrichtung wahlweise eine Anordnung von pH-Sensoren beinhaltet und wobei das Verfahren wahlweise ferner das Sequenzieren unter Verwendung der Halbleitersequenzierungsvorrichtung umfasst.

13. Verfahren zum Laden von Kügelchen auf ein Sensorsubstrat, das Verfahren umfassend:
Zufügen einer Suspension, die Nukleinsäurekügelchen enthält, zu einer Durchflusszelle, die über einem Sensorsubstrat definiert ist, wobei das Sensorsubstrat eine Vielzahl von Mulden umfasst, wobei sich die Kügelchen mindestens teilweise in der Vielzahl von Mulden absetzen;
Strömenlassen einer Gas/Flüssigkeitsgrenzschicht durch die Durchflusszelle und über das Sensorsubstrat;
Verdampfen von Flüssigkeit von der Durchflusszelle für eine Dauer von mindestens 15 Sekunden und nicht länger als 30 Minuten mit einer Rate im Bereich von 100 µl/min bis 100 ml/min; und
Zuführen einer Kondensationslösung durch die Durchflusszelle.

14. Verfahren nach Anspruch 13, wobei die Kondensationslösung ein Magnesiumsalz und ein Polyethylenglykolpolymer beinhaltet.

15. Verfahren nach Anspruch 13 oder Anspruch 14, wobei das Strömenlassen einer Gas/Flüssigkeitsgrenzschicht Strömenlassen eines Schaums durch die Durchflusszelle beinhaltet.

## Revendications

1. Procédé de chargement de billes sur un substrat de capteur, le procédé consistant à :
appliquer une suspension comportant des billes à une cellule d'écoulement définie par-dessus un substrat de capteur, le substrat de capteur comprenant une pluralité de puits, les billes se déposant au moins partiellement dans la pluralité de puits ;
retirer le liquide de la cellule d'écoulement ;
faire évaporer le liquide de la cellule d'écoulement ; et
appliquer une solution hydratante à la cellule d'écoulement.

2. Procédé selon la revendication 1, l'application de la suspension comportant le fait de soumettre à centrifugation le substrat de capteur en présence des billes.

3. Procédé selon la revendication 1 ou la revendication 2, comprenant en outre le fait de faire écouler une ou plusieurs bulles à travers la cellule d'écoulement et par-dessus le substrat de capteur avant de retirer le liquide de la cellule d'écoulement.

4. Procédé selon l'une quelconque des revendications 1-3, l'évaporation du liquide de la cellule d'écoulement comprenant l'extraction de gaz à travers la cellule d'écoulement pour une durée d'au moins 15 secondes et au maximum de 30 minutes.

5. Procédé selon l'une quelconque des revendications 1-4, l'évaporation du liquide de la cellule d'écoulement comprenant l'extraction de gaz à travers la cellule d'écoulement à un débit compris dans une plage allant de 100 µL/min à 100 mL/min.

6. Procédé selon l'une quelconque des revendications 1-5, comprenant en outre l'application d'une solution de condensation par-dessus le substrat de capteur.

7. Procédé selon la revendication 6, l'application de la solution de condensation comportant l'application de la solution de condensation après extraction de gaz à travers la la cellule d'écoulement.

8. Procédé selon la revendication 6, l'application de la solution de condensation comportant l'application de la solution de condensation avant l'évaporation du liquide de la cellule d'écoulement.

9. Procédé selon la revendication 6, la solution de condensation comportant un agent de condensation, facultativement l'agent de condensation comportant du magnésium, un polymère de polyéthylène glycol ou une combinaison de ces derniers.

10. Procédé selon l'une quelconque des revendications 1-9, comprenant en outre l'application d'une solution enzymatique à travers la cellule d'écoulement et par-dessus le substrat de capteur.

11. Procédé selon la revendication 10, comprenant en outre le retrait du liquide de la cellule d'écoulement et l'évaporation du liquide de la cellule d'écoulement après application de la solution enzymatique.

12. Procédé selon l'une quelconque des revendications 1-11, le substrat de capteur comportant un dispositif de séquençage à semi-conducteurs, facultativement le dispositif de séquençage à semi-conducteurs comportant une série de capteurs de pH, et facultativement le procédé consistant en outre à séquencer à l'aide du dispositif de séquençage à semi-conducteurs.

13. Procédé de chargement de billes sur un substrat de capteur, le procédé consistant à :
appliquer une suspension comportant des billes d'acide nucléique sur une cellule d'écoulement définie par-dessus un substrat de capteur, le substrat de capteur comprenant une pluralité de puits, les billes se déposant au moins partiellement dans la pluralité de puits ;
faire écouler une interface gaz/liquide à travers la cellule d'écoulement et par-dessus le substrat de capteur ;
faire évaporer le liquide de la cellule d'écoulement pour une durée d'au moins 15 secondes et d'au maximum de 30 minutes à un débit compris dans une plage allant de 100 µL/min à 100 mL/min ; et
appliquer une solution de condensation à travers la cellule d'écoulement.

14. Procédé selon la revendication 13, la solution de condensation comportant un sel de magnésium et un polymère de polyéthylène glycol.

15. Procédé selon la revendication 13 ou la revendication 14, l'écoulement de l'interface gaz/liquide comportant l'écoulement d'une mousse à travers la cellule d'écoulement.
